# FASCICULE DE BREVET EUROPEEN

(11) **EP 3 852 709 B1**
(45) Date de publication et mention de la délivrance du brevet: **09.08.2023**
(21) Numéro de dépôt: 19790653.0
(22) Date de dépôt: 17.09.2019
(51) Int. Cl.: A61H 1/02, A61H 3/00, B25J 9/00, B25J 9/10, A61F 5/01, A63B 21/068, A63B 23/04, A63B 21/00

(54) **DISPOSITIF ET PROCEDE D'ASSISTANCE A LA MOBILISATION D'UNE ARTICULATION**
VORRICHTUNG UND VERFAHREN ZUR UNTERSTÜTZUNG DER MOBILISIERUNG EINES GELENKS
DEVICE AND METHOD FOR ASSISTING WITH MOBILIZING A JOINT

(30) Priorité: 19.09.2018 FR 1858459
(43) Date de publication de la demande: 28.07.2021
(73) Titulaire: Deville, Lilas, 86180 Buxerolles (FR); Gastebois, Jérémy, 86180 Buxerolles (FR)
(72) Inventeur: Deville, Lilas, 86180 Buxerolles (FR); Gastebois, Jérémy, 86180 Buxerolles (FR)
(74) Mandataire: Ipside
(86) Numéro de dépôt international: PCT/FR2019/052158
(87) Numéro de publication internationale: WO 2020/058621

(56) Documents cités:
- WO-A1-2014/109799
- WO-A1-2018/135402
- WO-A2-2016/089466
- US-A- 1 308 675

## Description

### Domaine technique

L'invention se situe dans le domaine des dispositifs exosquelettes souples d'assistance à la mobilisation d'au moins une articulation d'un corps, et plus particulièrement dans celui des exosquelettes souples d'assistance à la mobilisation par câble d'au moins une articulation.

L'invention concerne également un produit programme d'ordinateur pour l'exécution des étapes d'un procédé de contrôle d' un dispositif selon l'invention.

### État de la technique antérieure

De la demande internationale WO 2017/026943, on connaît un dispositif d'assistance par câble à la mobilisation d'une articulation d'un corps, et ce selon un mouvement de flexion ou un mouvement d'extension du coude. Le dispositif présente deux câbles, l'un relié à la partie postérieure de l'avant-bras, l'autre relié à la partie antérieure de l'avant-bras.

Dans la présente description, le système de référence en anatomie est utilisé, lorsque le sujet est dans la position de standard anatomique humaine, position dite de Paul Poirier.

Le dispositif selon l'art antérieur n'est pas totalement satisfaisant.

Ce dispositif peut ne pas convenir à de nombreuses personnes lorsqu'on essaie de l'appliquer à une articulation telle que celle du genou qui articule le fémur au tibia et à la patella.

En effet, certaines personnes peuvent souffrir de valgus ou varus. Dans le cas du genou, le varus correspond à une rotation externe du fémur, ce qui éloigne latéralement l'articulation du genou se traduisant visuellement par des jambes arquées. À l'opposé, le valgus est une rotation interne du fémur qui rapproche le genou de l'axe longitudinal, avec pour résultat des genoux dits «cagneux» ou «en X». On comprend que l'application du dispositif selon l'art antérieur à une personne souffrant de valgus ou varus conduirait à générer un couple de rotation à la cheville selon l'axe antéro-postérieur, ce qui n'est pas souhaité.

En outre, il est difficile de déterminer précisément l'axe de rotation du genou, qui n'est pas toujours colinéaire avec l'axe transversal. Aussi, l'utilisation du dispositif selon l'art antérieur pourrait conduire à appliquer un couple de rotation à l'articulation du genou qui présente une composante non colinéaire avec l'axe de l'articulation, ce qui pourrait être douloureux et même dangereux. Pour connaître la position de l'axe de rotation d'une articulation, les biomécaniciens peuvent avoir recours à des techniques de capture de mouvement, qui permettent d'arriver à des précisions raisonnables, au prix d'une mise en oeuvre compliquée. Ces techniques ne sont donc pas envisageables pour un exosquelette se voulant facilement adaptable. De plus la position de l'axe de rotation d'une articulation peut se déplacer pendant le mouvement, c'est par exemple le cas du genou.

Dans le cadre de l'articulation du genou, on comprend donc que le dispositif proposé par l'art antérieur n'est pas totalement satisfaisant pour certaines personnes.

En réalité, le dispositif selon l'art antérieur n'est pas non plus totalement satisfaisant dans le cadre de son utilisation pour un coude. En effet, outre les mouvements de flexion et d'extension de l'avant-bras par rapport au bras, les articulations du coude permettent un deuxième degré de liberté qu'est la prono-supination, qui est la rotation de l'avant-bras autour de son axe longitudinal.

Encore d'autres articulations sont concernées. Par exemple, l'articulation de la cheville, qui relie la jambe et le pied, peut présenter une éversion, ce qui correspond à une rotation externe latérale. Dans le cas de l'os du talon (le calcanéum), la plante de pied est orientée latéralement. À l'inverse, l'inversion est une rotation interne médiale. La plante de pied est alors orientée médialement. D'autres mouvements du pied sont possibles, du fait des articulations propres au pied. Le positionnement d'un pied par rapport à une jambe aux seuls moyens de deux câbles est donc très approximatif.

Par ailleurs, dans le corps humain, les muscles responsables des mouvements fonctionnent par paire, et sont appelés agonistes et antagonistes. Le quadriceps fémoral et les ischiojambiers pour le genou ou le biceps et le triceps pour le coude sont deux exemples de ces paires de muscles.

Lorsque le muscle agoniste est contracté pour réaliser un mouvement, par exemple une flexion du genou, son muscle antagoniste est étiré afin de ne pas gêner le mouvement. Les rôles sont inversés quand le mouvement inverse est effectué. Dans le cadre du genou, le quadriceps se contracte et les ischiojambiers s'étirent pendant l'extension du genou. Par opposition, le quadriceps s'étire et les ischiojambiers se contractent pendant la flexion du genou.

Le dispositif selon l'art antérieur prévoit de n'utiliser qu'un seul moteur pour actionner les deux câbles, alternativement. Autrement dit, une seule direction de déplacement du bras ne peut être sollicitée à la fois lorsqu'un dispositif selon l'art antérieur est utilisé. Cela génère un problème de prétension du câble qui n'est pas actionné. Par ailleurs, il n'est alors pas possible d'appliquer un effort selon deux sens opposés. En conclusion, il n'est possible d'utiliser ce type de dispositif que pour un nombre limité de guidages en position de l'articulation.

On connaît aussi le document US 1 308 675 A qui ne présente qu'une paire de câbles qui se séparent en deux au-dessus des hanches. Les deux câbles passant devant le genou sont commandés simultanément par l'unité d'actionnement située dans le dos. Les deux câbles situés derrière le genou sont actionnés simultanément de manière passive par les ressorts situés sur l'avant du torse. On ne peut pas contrôler la direction du couple articulaire.

On connaît également le document WO 2016/089466 A2, WO 2018/135402 A1, WO 2014/109799 A1 qui ne présentent qu'un câble (ou un câble divisé en deux) passant devant la hanche et derrière le genou, ou au niveau de la hanche et de la cheville et présente au moins les mêmes inconvénients que US 1 308 675 A.

### Exposé de l'invention

Un but de l'invention est notamment de remédier à tout ou partie des inconvénients précités.

Selon un premier aspect de l'invention, il est proposé un dispositif d'assistance à la mobilisation d'une articulation reliant un segment (c.-à-d. un os) supérieur et un segment inférieur d'une personne, comportant :
- un premier élément de guidage souple configuré pour être positionné sur ou à proximité de l'un des segments, le premier élément de guidage présentant une première paire de passages de câble, un passage de câble, respectivement l'autre passage de câble de la première paire de passages de câble du premier élément de guidage étant disposé du côté de la face postérieure, respectivement du côté de la face antérieure, dudit segment lorsque la personne porte le premier élément de guidage,
- un deuxième élément de guidage souple configuré pour être positionné sur ou à proximité de l'autre des segments, le deuxième élément de guidage présentant une première paire de passages de câble, un passage de câble, respectivement l'autre passage de câble, de la première paire de passages de câble étant disposé du côté de la face postérieure, respectivement du côté de la face antérieure, dudit segment lorsque la personne porte le deuxième élément de guidage, le deuxième élément de guidage comportant en outre une paire de points d'ancrage, un point d'ancrage, respectivement l'autre point d'ancrage, de la paire de points d'ancrage étant disposé du côté de la face antérieure, respectivement du côté de la face postérieure, dudit segment,
- une pièce de support souple agencée pour être portée par la personne et sur laquelle sont disposés une paire d'actionneurs et une paire d'enrouleurs, chacun des actionneurs de la paire d'actionneurs étant associé à un enrouleur de la paire d'enrouleurs et étant agencé pour faire tourner l'enrouleur associé selon le sens d'enroulement et le sens de déroulement,
- une paire de câbles comportant un premier câble, respectivement un deuxième câble s'étendant depuis l'un des enrouleurs, respectivement l'autre des enrouleurs, de la paire d'enrouleurs, jusqu'à l'un des points d'ancrage, respectivement l'autre des points d'ancrage, de l'élément d'ancrage souple, en passant d'une part par un passage de câble, respectivement l'autre passage de câble, de la première paire de passages de câble du premier élément de guidage et d'autre part par un passage de câble, respectivement l'autre passage de câble, de la première paire de passages de câble du deuxième élément de guidage,
- un contrôleur configuré pour commander indépendamment chacun des actionneurs de la paire d'actionneurs, dans lequel:
- le premier élément de guidage souple comporte en outre une deuxième paire de passages de câble, un passage de câble, respectivement l'autre passage de câble, de ladite deuxième paire de passages du premier élément de guidage étant disposé du côté de la face postérieure, respectivement du côté de la face antérieure, dudit segment lorsque la personne porte le premier élément de guidage,
- le deuxième élément de guidage souple comporte en outre une deuxième paire de passages de câble, un passage de câble, respectivement l'autre passage de câble, de ladite deuxième paire de passages du deuxième élément de guidage étant disposé du côté de la face postérieure, respectivement du côté de la face antérieure, dudit segment lorsque la personne porte le deuxième élément de guidage, le deuxième élément de guidage pouvant en outre comporter une deuxième paire de points d'ancrage, un point d'ancrage, respectivement l'autre point d'ancrage, de la deuxième paire de points d'ancrage étant disposé du côté de la face antérieure, respectivement du côté de la face postérieure, du segment inférieur lorsque la personne porte le deuxième élément de guidage,

Le dispositif comporte en outre une deuxième paire d'actionneurs et une deuxième paire d'enrouleurs, chacun des actionneurs de la deuxième paire d'actionneurs étant associé à un enrouleur de la deuxième paire d'enrouleurs et étant agencé pour faire tourner l'enrouleur associé selon le sens d'enroulement et le sens de déroulement, les deuxièmes paires d' actionneur et d'enrouleur étant fixées sur la pièce de support souple, et une deuxième paire de câbles comportant un premier câble, respectivement un deuxième câble, s'étendant depuis l'un des enrouleurs, respectivement l'autre des enrouleurs, de la deuxième paire d'enrouleurs, jusqu'à l'un des points d'ancrage, respectivement l'autre des points d'ancrage, du deuxième élément de guidage, en passant d'une part par un passage de câble, respectivement l'autre passage de câble, de la deuxième paire de passages de câble du premier élément de guidage et d'autre part par un passage de câble, respectivement l'autre passage de câble, de la deuxième paire de passages de câble du deuxième élément de guidage, le contrôleur étant en outre configuré pour commander, indépendamment, chacun des actionneurs de la deuxième paire d'actionneurs.

Avantageusement, le contrôleur est configuré pour commander les actionneurs de la paire d'actionneurs selon une loi de commande prédéterminée.

Chacun des câbles de la première et/ou, le cas échéant, de la deuxième, paire de câbles peut être un câble souple pouvant coulisser dans une gaine souple. Le câble souple peut être du type des lignes utilisées dans les sports aériens tels que le parapente, pour des questions de facilité de mise en oeuvre. Ce type de cordelette supporte de très fortes charges, ne se déforme quasiment pas et est facile à mettre en oeuvre. Il présente l'avantage d'être très souple, et peut s'enrouler facilement autour de bobines de faible diamètre. Le câble souple peut aussi être fait d'acier torsadé, d'un diamètre judicieusement choisi.

Les deux extrémités de chaque câble peuvent être munies d'un embout serti ou vissé, et sont reliées, respectivement, à l'un des enrouleurs et son point d'ancrage. La gaine, peut être composée d'un ressort à spires jointives, et peut être enveloppée d'une gaine de coton ou de plastique.

Lorsque le premier élément de guidage souple et le deuxième élément de guidage souple sont disposés sur la personne, chacun des câbles présente un point remarquable, déterminé comme le point le plus proche d'une position estimée de l'axe de rotation de l'articulation.

Selon une possibilité, la direction formée par les points remarquables de la première paire de câble, et celle formée par les deux points remarquables de la deuxième paire de câble sont sensiblement orthogonales.

Avantageusement, les directions forment un angle de 45° avec l'axe transversal et l'axe antéro-postérieur, lorsque la personne porte l'élément d'ancrage (dit autrement, gauche droite devant derrière).

De manière encore plus avantageuse, il est proposé de mettre en oeuvre plus de deux paires de câbles. On obtient alors une redondance d'actionnement. Dans le cadre de grosses articulations, telles que le dos, par exemple, la redondance d'actionnement permet d'utiliser un plus grand nombre de moteurs moins puissants et de plus de mieux répartir la charge sur le patient.

Selon une particularité, le dispositif selon le premier aspect de l'invention comporte un moyen de détermination de l'orientation du segment supérieur et/ou du segment inférieur par rapport à l'axe longitudinal.

De préférence, le dispositif selon le premier aspect de l'invention comporte en outre un moyen de détermination du couple articulaire.

Selon une particularité, le dispositif selon le premier aspect de l'invention comporte un moyen de détermination de l'orientation du segment supérieur et/ou du segment inférieur par rapport à l'axe longitudinal.

Le moyen de détermination du couple articulaire ainsi que l'orientation des segments par rapport à l'axe longitudinal (orientation par rapport à la gravité) peut être réalisée à partir d'un moyen de détermination des deux angles formés entre le segment supérieur et le segment inférieur.

À titre d'exemple, le moyen de détermination des deux angles peut être réalisé au moyen de deux inclinomètres bidirectionnels, placés l'un sur le segment supérieur et l'autre sur le segment inférieur. Les deux inclinomètres permettent de mesurer les angles du segment supérieur et du segment inférieur dans les plans sagittal et transversal. Ces capteurs d'angles permettent de mesurer la disposition géométrique des segments et d'en déduire le couple de gravité statique sur le genou. La mesure de la disposition géométrique des segments permet une reconstruction estimée de la position de l'axe de rotation, et on peut actionner les deux paires de câbles afin de générer un couple qui soit le plus aligné possible avec cette reconstruction de l'axe de rotation.

Le moyen de détermination du couple articulaire peut comporter des centrales inertielles. Une détermination du couple articulaire peut alors prendre en compte des phénomènes dynamiques. On peut mesurer les accélérations subies par chacun des segments, et ainsi pouvoir en déduire les efforts qu'ils subissent. De cette manière il est possible de déterminer le couple exercé sur l'articulation, qui est la somme du couple de gravité et de toutes les autres contributions pouvant intervenir (inertie, etc...). Le couple de gravité peut être déduit de la position, qu'il est possible d'obtenir par deux intégrations successives des accélérations. L'utilisation de centrales inertielles permet une meilleure approximation de la reconstruction de l'axe de rotation.

Selon un mode de réalisation, il est prévu d'utiliser des capteurs d'effort placés au bout de chacun des câbles, et de mesurer la différence entre la tension de câbles mesurée par ces capteurs et le couple développé par les actionneurs, par exemple mesuré à partir de l'intensité dans les moteurs, ce qui détermine une mesure de «l'intention» du porteur de l'exosquelette. En effet, le couple actionneur représente le couple fourni par l'exosquelette et la mesure des capteurs permet de déterminer si la personne tire sur certains câbles ou en détend d'autres, ce qui permet de déduire le sens d'évolution du mouvement.

À titre d'exemple, le dispositif selon le premier aspect de l'invention, ou l'un ou plusieurs de ses perfectionnements, comporte un moyen de détection d'un appui du segment inférieur sur un sol ou un bâti.

Toujours à titre d'exemple, le dispositif selon le premier aspect de l'invention, ou l'un ou plusieurs de ses perfectionnements, présente une chaîne de transmission entre un moteur et un point d'ancrage qui comporte un palan.

Selon un deuxième aspect de l'invention, il est proposé un produit programme d'ordinateur pour l'exécution des étapes d'un procédé d'assistance, non thérapeutique, de manière préférentielle dans le cadre d'activité de loisir ou sportive ou encore des applications à l'assistance à la personne à domicile (on pense par exemple aux personnes âgées, ou ayant des difficultés de mobilité), à la mobilisation d'une articulation reliant un segment supérieur et un segment inférieur, comportant un dispositif à la mobilisation de l'articulation selon le premier aspect de l'invention, ou l'un ou plusieurs de ses perfectionnements.

Des capteurs d'effort peuvent être utilisés pour contrôler le dispositif selon l'invention.

Selon une possibilité, le procédé d'assistance peut comprendre en outre une étape d'estimation du couple articulaire à partir d'informations provenant de capteurs équipant le dispositif, et une étape de calcul et d'application d'un couple d'assistance, correspondant à un pourcentage d'assistance prédéterminé.

Avantageusement, le procédé d'assistance peut en outre comprendre une étape de de filtrage, par exemple de type passe bas, des informations provenant des capteurs.

Le procédé d'assistance peut comporter une étape de filtrage, dans lequel la loi de commande du dispositif est déterminée à partir d'une trajectoire prédéterminée.

Le procédé d'assistance peut comporter un suivi de l'évolution de l'écart de position en valeur absolue entre d'une part la position réelle du segment inférieur à un instant donné et d'autre part la consigne de position de la trajectoire déterminée audit instant donné.

Le procédé d'assistance peut comporter une application par les actionneurs du dispositif, d'une part d'une force réactive lorsque ledit écart augmente et d'autre part d'une force active lorsque ledit écart diminue.

Selon le deuxième aspect de l'invention, il est proposé que le produit programme d'ordinateur, chargeable directement dans la mémoire interne d'un ordinateur, comprenne des portions de code de logiciel pour l'exécution des étapes du procédé de visualisation selon le deuxième aspect de l'invention, ou l'un ou plusieurs de ses perfectionnements, lorsque ledit programme est exécuté sur un ordinateur.

Le produit programme d'ordinateur peut comporter des moyens de réglages du procédé d'assistance, par exemple au moyen d'une interface utilisateur, de préférence affichée sur un écran d'un ordinateur ou d'un smartphone.

Les réglages peuvent par exemple être des réglages d'intensité d'assistance, de mode de fonctionnement.

### Description des figures

D'autres avantages et particularités de l'invention apparaîtront à la lecture de la description détaillée de mises en oeuvre et de modes de réalisation nullement limitatifs, au regard de dessins annexés sur lesquels :
- la figure 1 représente schématiquement, trois vues en perspectives et partielles d'une jambe droite, respectivement depuis la gauche, l'arrière, et la droite, équipée d'un dispositif selon un mode de réalisation de l'invention,
- la figure 2 représente schématiquement le positionnement de points de passages remarquables de câble du dispositif représenté sur la figure 1, selon un plan transverse ; elle est une vue en coupe du genou (et du dispositif) selon le plan transverse.

### Description de modes de réalisation

Les modes de réalisation décrits ci-après n'étant nullement limitatifs, on pourra notamment considérer des variantes de l'invention ne comprenant qu'une sélection de caractéristiques décrites, par la suite isolées des autres caractéristiques décrites, si cette sélection de caractéristiques est suffisante pour conférer un avantage technique ou pour différencier l'invention telle que décrite dans les revendications, par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

Sur les figures un élément apparaissant sur plusieurs figures conserve la même référence.

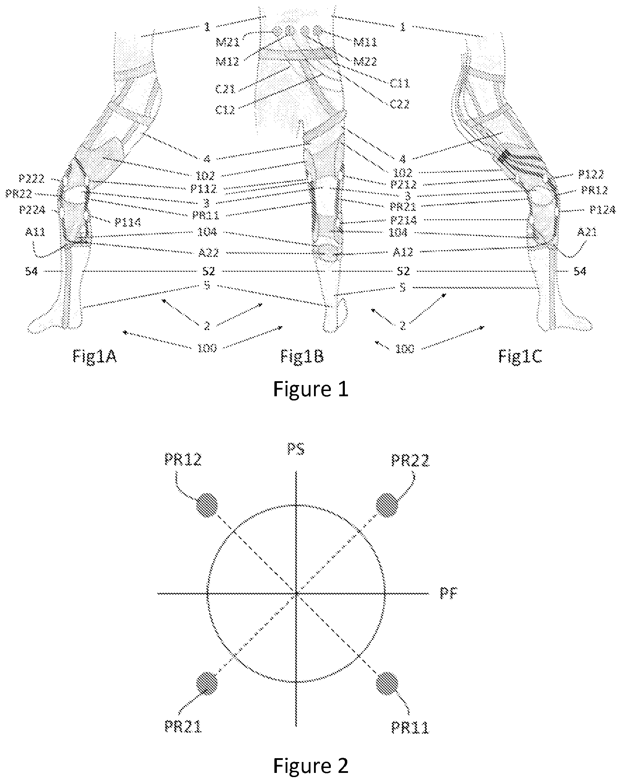

Les figures 1A, 1B et 1C représentent une partie d'une personne 1, et nomment une jambe droite 2, respectivement selon une vue latérale gauche, une vue postérieure et une vue latérale droite.

La jambe 2 est équipée d'un dispositif 100 d'assistance à la mobilisation d'une articulation 3 reliant un segment supérieur 4 et un segment inférieur 5.

Selon le mode de réalisation illustré, l'articulation 3 est un genou droit, le segment supérieur 4, respectivement le segment inférieur 5, étant formé d'un fémur, respectivement du tibia et de la patella.

Le dispositif 100 comporte :
- un premier élément de guidage 102 souple configuré pour être positionné sur ou à proximité du segment supérieur 4 de la personne, lorsque la personne porte le premier élément de guidage,
- un deuxième élément de guidage 104 souple configuré pour être positionné sur ou à proximité du segment inférieur 5 de la personne 1, lorsque la personne porte le deuxième élément de guidage,
- une pièce de support souple 106 (non représentée) agencée pour être portée par la personne 1.

Le premier élément de guidage 102 comporte :
- une première paire de passages de câble, un passage de câble P112, respectivement l'autre passage de câble P122, de la première paire de passages de câble étant disposé du côté de la face postérieure 52, respectivement du côté de la face antérieure 54, dudit segment lorsque la personne porte le premier élément de guidage,
- une deuxième paire de passages de câble, un passage de câble P212, respectivement l'autre passage de câble P222, de la deuxième paire de passages de câble étant disposé du côté de la face postérieure 52, respectivement du côté de la face antérieure 54, dudit segment lorsque la personne porte le premier élément de guidage,

Le deuxième élément de guidage 104 d'ancrage souple comporte :
- une première paire de passages de câble, un passage de câble P114, respectivement l'autre passage de câble P124, de la première paire de passages de câble étant disposé du côté de la face postérieure 52, respectivement du côté de la face antérieure 54, dudit segment lorsque la personne porte le deuxième élément de guidage,
- une deuxième paire de passages de câble, un passage de câble P214, respectivement l'autre passage de câble P224, de la deuxième paire de passages de câble étant disposé du côté de la face postérieure 52, respectivement du côté de la face antérieure 54, dudit segment lorsque la personne porte le premier élément de guidage,
- une première paire de points d'ancrage, un point d'ancrage A11, respectivement l'autre point d'ancrage A12, de la première paire de points d'ancrage étant disposé du côté de la face antérieure 52, respectivement du côté de la face postérieure 54, dudit segment, lorsque la personne porte le deuxième élément de guidage 104,
- une deuxième paire de points d'ancrage, un point d'ancrage A21, respectivement l'autre point d'ancrage A22, de la deuxième paire de points d'ancrage étant disposé du côté de la face antérieure 52, respectivement du côté de la face postérieure 54, dudit segment, lorsque la personne porte le deuxième élément de guidage 104.

Deux paires d'actionneurs M11, M12 et M21, M22 de type motoréducteurs et deux paires d'enrouleurs (non représentés) sont disposés sur la pièce de support souple 106, chacun des actionneurs de la paire d'actionneurs étant associé à un enrouleur de la paire d'enrouleurs et étant agencé pour faire tourner l'enrouleur associé de type bobine selon le sens d'enroulement et le sens de déroulement.

Le dispositif 100 comporte en outre :
- deux paires de câbles C11, C12 et C21, C22,
- un contrôleur (non représenté) configuré pour commander chacun des actionneurs de la paire d'actionneurs.

La première paire de câbles C11, C12 comporte un premier câble C11, respectivement un deuxième câble C12, s'étendant depuis l'enrouleur relié à l'actionneur M11, respectivement l'autre enrouleur relié à l'actionneur M12, de chacune des paires d'enrouleurs, jusqu'à l'un des points d'ancrage A11, respectivement l'autre des points d'ancrage A12, du deuxième élément de guidage 104, en passant d'une part par un passage de câble P112, respectivement l'autre passage de câble P122, de la première paire de passages de câble du premier élément de guidage 102 et d'autre part par un passage de câble P114, respectivement l'autre passage de câble P124, de la première paire de passages de câble du deuxième élément de guidage 104.

La deuxième paire de câbles C21, C22 comporte un premier câble C21, respectivement un deuxième câble C22, s'étendant depuis l'enrouleur relié à l'actionneur M21, respectivement l'autre enrouleur relié à l'actionneur M22, jusqu'à l'un des points d'ancrage A21, respectivement l'autre des points d'ancrage A22, du deuxième élément de guidage 104, en passant d'une part par un passage de câble P212, respectivement l'autre passage de câble P222, de la deuxième paire de passages de câble du premier élément de guidage 102 et d'autre part par un passage de câble P214, respectivement l'autre passage de câble P224, de la deuxième paire de passages de câble du deuxième élément de guidage 104.

Lorsque le premier élément de guidage souple et le deuxième élément de guidage souple sont disposés sur la personne, chacun des câbles présente un point remarquable, déterminé comme le point le plus proche de l'axe de rotation de l'articulation.

La figure 2 illustre une coupe du genou de la jambe droite vue de dessous, dans le plan transverse. Le plan sagittal PS et le plan frontal PF y sont représentés, ainsi que les quatre points remarquables respectivement PR11, PR12, PR21 et PR22 par lesquels passent, respectivement, les câbles C11, C12, C21 et C22.

Comme l'illustre la figure 2, les directions formées sont sensiblement orthogonales. La superposition des couples produits par les deux paires de câbles permet de générer tout couple perpendiculaire au plan transverse.

Plus précisément, les directions formées par les points remarquables PR11, PR12 de la première paire de points remarquables, respectivement les deux points remarquables PR21, PR22 de la deuxième paire de points remarquables, forment un angle de 45° avec l'axe transversal et l'axe antéro-postérieur, lorsque la personne porte l'élément d'ancrage. Cette disposition des câbles est plus confortable que si deux câbles étaient disposés dans le plan frontal, ce qui serait particulièrement inconfortables. En effet, l'un des deux câbles d'une paire serait situé entre les jambes, gênant ainsi la marche et l'un des deux câbles de l'autre paire serait situé derrière le genou, ce qui serai gênant pour replier complètement le genou.

On utilise le positionnement des passages de câbles pour garantir une distance entre chaque câble et une position estimée du centre de l'articulation. On utilise également les passages de telle sorte que les portions libres des câbles tendus soient réparties le plus régulièrement possible autour de la circonférence du genou. Les câbles peuvent être insérés dans des gaines de type Bowden afin de limiter au maximum la déformation des câbles sous tension. Les imprécisions de positionnement de passages de câbles peuvent être compensées aux moyens d'une loi de commande robuste.

Le contrôleur est configuré pour commander les actionneurs de la paire d'actionneurs selon une loi de commande prédéterminée.

### Mise en oeuvre d'un dispositif d'assistance à la mobilisation de l'articulation

Avec un dispositif selon l'invention, selon une première possibilité, il est proposé un produit programme d'ordinateur pour l'exécution des étapes d'un procédé d'assistance au mouvement, comportant :
- une étape d'estimation du couple articulaire à partir des informations des capteurs (statique ou dynamique)
- une étape de calcul et d'application d'un couple d'assistance, correspondant à un pourcentage d'assistance choisi en amont, par exemple par un médecin.

Selon une possibilité, il est ainsi proposé une solution non médicamenteuse, permettant de retrouver ou de conserver une activité physique et ainsi de renforcer les articulations touchées en procurant un niveau d'assistance adapté au cas du patient touché. Il est ainsi possible de retarder le recours au traitement médical ou l'alléger, et retarder l'utilisation de prothèses dans les cas les plus sévères..

Selon une possibilité, il est ainsi proposé une solution permettant d'apporter une assistance à la mobilité dans le cadre de pratiques de loisirs ou sportives, ainsi qu'au quotidien pour les personnes ayant des difficultés de mobilité.

Selon une deuxième possibilité, il est proposé un procédé d'apprentissage d'un mouvement, ne faisant pas partie de l'invention, dans lequel la loi de commande du dispositif est déterminée à partir d'une trajectoire prédéterminée.

Le procédé comprend un suivi de l'évolution de l'écart de position en valeur absolue entre d'une part la position réelle du segment inférieur à un instant donné et d'autre part la consigne de position de la trajectoire déterminée audit instant donné, et une application par les actionneurs du dispositif, d'une part d'une force réactive lorsque ledit écart augmente et d'autre part d'une force active lorsque ledit écart diminue.

De manière plus précise, le procédé ne faisant pas partie de l'invention, comprend :
- une étape d'enregistrement d'un mouvement à apprendre,
- une étape de génération du champ de force correspondant au mouvement à apprendre,
- une étape d'estimation de la valeur absolue de l'écart de position,
   o si la valeur absolue de l'écart de position augmente, une génération d'un champ de force supplémentaire s'opposant au mouvement de la personne,
   o si la valeur absolue de l'écart de position diminue ou devient nul, une génération d'un champ de force, éventuellement nul, assistant, ou ne gênant pas, le mouvement.

Pour l'étape d'enregistrement du mouvement à apprendre, on envisage d'équiper une personne maîtrisant le geste à apprendre d'un dispositif selon l'invention. Il serait alors en mode «enregistrement» et mesurerait passivement la trajectoire suivie. Cette trajectoire serait utilisée pour créer un champ de force tridimensionnel, dont la fonction serait d'encourager le « bon » mouvement et de décourager le « mauvais » mouvement. Quand la personne équipée de l'exosquelette et devant apprendre le mouvement s'écarte de la trajectoire à suivre, l'exosquelette lui oppose une résistance augmentant avec l'écart à la trajectoire. Au contraire, quand l'individu suit une trajectoire proche de la trajectoire à suivre, l'exosquelette n'exerce aucune résistance, ou même propose une assistance adaptée.

Ce même principe pourrait être utilisé pour des applications de rééducation, en mettant plus l'accent sur l'assistance apportée au patient.

Par ailleurs, en plus de guider le mouvement pour obtenir une trajectoire prédéterminée, par exemple une trajectoire selon un mouvement de marche, le procédé pourrait guider pour obtenir un mouvement dans le cadre de pratiques sportives : exercice de «montée aux anneaux» en gymnastique, mouvement de golf, mouvement de natation.

### Élimination des tremblements

Avec un dispositif selon l'invention, il est aussi possible de proposer un procédé de réduction des tremblements, ne faisant pas partie de l'invention.

Certaines maladies comme la maladie de Parkinson provoquent des tremblements incontrôlés chez les personnes touchées. On peut envisager de programmer le contrôleur du dispositif selon l'invention avec une assistance similaire à celle décrite dans le cadre de la première possibilité, et d'y adjoindre un filtre passe-bas bien ajusté permettant de filtrer les tremblements parasites du sujet.

Bien sûr, l'invention n'est pas limitée aux exemples qui viennent d'être décrits et de nombreux aménagements peuvent être apportés à ces exemples sans sortir du cadre de l'invention telle que décrite dans les revendications.

De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres et que se limitent aux revendications.

## Revendications

1. Dispositif (100) d'assistance à la mobilisation d'une articulation (3) reliant un segment supérieur (4) et un segment inférieur (5) d'une personne (1), comportant :
- un premier élément de guidage (102) souple configuré pour être positionné sur ou à proximité de l'un des segments (4), le premier élément de guidage présentant une première paire de passages de câble fixés sur ledit premier élément de guidage, un passage de câble (P112), respectivement l'autre passage de câble (P122), de la première paire de passages de câble du premier élément de guidage étant disposé du côté de la face postérieure (52), respectivement du côté de la face antérieure (54), dudit segment lorsque la personne porte le premier élément de guidage,
- un deuxième élément de guidage (104) souple configuré pour être positionné sur ou à proximité de l'autre des segments (5), le deuxième élément de guidage présentant une première paire de passages de câble fixés sur ledit deuxième élément de guidage, un passage de câble (P114), respectivement l'autre passage de câble (P124), de la première paire de passages de câble du deuxième élément de guidage étant disposé du côté de la face postérieure (52), respectivement du côté de la face antérieure (54), dudit segment lorsque la personne porte le deuxième élément de guidage, le deuxième élément de guidage comportant en outre une paire de points d'ancrage, un point d'ancrage (A11), respectivement l'autre point d'ancrage (A12), de la paire de points d'ancrage étant disposé du côté de la face antérieure (54), respectivement du côté de la face postérieure (52), dudit segment, lorsque la personne porte le deuxième élément de guidage,
- une pièce de support souple agencée pour être portée par la personne et sur laquelle sont disposés une paire d'actionneurs (M11, M12) et une paire d'enrouleurs, chacun des actionneurs de la paire d'actionneurs étant associé à un enrouleur de la paire d'enrouleurs et étant agencé pour faire tourner l'enrouleur associé selon le sens d'enroulement et le sens de déroulement,
- une paire de câbles (C11, C12) comportant un premier câble (C11), respectivement un deuxième câble (C12) s'étendant depuis l'un des enrouleurs, respectivement l'autre des enrouleurs, de la paire d'enrouleurs, jusqu'à l'un des points d'ancrage, respectivement l'autre des points d'ancrage, du deuxième élément de guidage, en passant d'une part par un passage de câble, respectivement l'autre passage de câble, de la première paire de passages de câble du premier élément de guidage et d'autre part par un passage de câble, respectivement l'autre passage de câble, de la première paire de passages de câble du deuxième élément de guidage,
- un contrôleur configuré pour commander indépendamment chacun des actionneurs de la paire d'actionneurs,
dans lequel :
- le premier élément de guidage (102) souple comporte en outre une deuxième paire de passages de câble fixés sur ledit premier élément de guidage, un passage de câble (P212), respectivement l'autre passage de câble (P222), de ladite deuxième paire de passages de câbles du premier élément de guidage étant disposé du côté de la face postérieure (52), respectivement du côté de la face antérieure (54), dudit segment lorsque la personne porte le premier élément de guidage,
- le deuxième élément de guidage (104) souple comporte en outre une deuxième paire de passages de câble fixés sur ledit deuxième élément de guidage, un passage de câble (P214), respectivement l'autre passage de câble (P224), de ladite deuxième paire de passages de câbles du deuxième élément de guidage étant disposé du côté de la face postérieure (52), respectivement du côté de la face antérieure (54), dudit segment lorsque la personne porte le deuxième élément de guidage, le deuxième élément de guidage comportant en outre une deuxième paire de points d'ancrage, un point d'ancrage (A21), respectivement l'autre point d'ancrage (A22), de la deuxième paire de points d'ancrage étant disposé du côté de la face antérieure (54), respectivement du côté de la face postérieure (52), du segment inférieur (5) de la personne (1) lorsque la personne porte le deuxième élément de guidage,
le dispositif comportant en outre :
- une deuxième paire d'actionneurs (M21, M22) et une deuxième paire d'enrouleurs, chacun des actionneurs de la deuxième paire d'actionneurs étant associé à un enrouleur de la deuxième paire d'enrouleurs et étant agencé pour faire tourner l'enrouleur associé selon le sens d'enroulement et le sens de déroulement, les deuxièmes paires d'actionneur et d'enrouleur étant fixées sur la pièce de support souple,
- une deuxième paire de câbles (C21, C22) comportant un premier câble (C21), respectivement un deuxième câble (C22), s'étendant depuis l'un des enrouleurs, respectivement l'autre des enrouleurs, de la deuxième paire d'enrouleurs, jusqu'à l'un des points d'ancrage, respectivement l'autre des points d'ancrage, du deuxième élément de guidage, en passant d'une part par un passage de câble, respectivement l'autre passage de câble, de la deuxième paire de passages de câble du premier élément de guidage et d'autre part par un passage de câble, respectivement l'autre passage de câble, de la deuxième paire de passages de câble du deuxième élément de guidage,
le contrôleur étant en outre configuré pour commander indépendamment chacun des actionneurs de la deuxième paire d'actionneurs.

2. Dispositif selon la revendication précédente, dans lequel le contrôleur est configuré pour commander les actionneurs de la paire d'actionneurs selon une loi de commande prédéterminée.

3. Dispositif selon la revendication précédente, dans lequel, lorsque le premier élément d'ancrage et le deuxième élément d'ancrage sont disposés sur la personne, chacun des câbles présente un point remarquable, déterminé comme le point le plus proche d'une position estimée de l'axe de rotation de l'articulation, et la direction formée par les deux points remarquables (PR11, PR12) de la première paire de câble et la direction formée par les deux points remarquables (PR21, PR22) de la deuxième paire de câble sont sensiblement orthogonales.

4. Dispositif selon la revendication précédente, dans lequel, les directions forment un angle de 45° avec l'axe transversal et l'axe antéro-postérieur.

5. Dispositif selon l'une quelconque des revendications précédentes, comportant plus de deux paires de câbles.

6. Dispositif selon l'une quelconque des revendications précédentes, comportant un moyen de détermination du couple articulaire.

7. Dispositif selon l'une quelconque des revendications précédentes, comportant un moyen de détermination de l'orientation du segment supérieur et/ou du segment inférieur par rapport à l'axe longitudinal.

8. Dispositif selon l'une quelconque des revendications précédentes, comportant un moyen de détection d'un appui du segment inférieur sur un sol ou un bâtit.

9. Dispositif selon l'une quelconque des revendications précédentes, dans lequel une chaîne de transmission entre un moteur et un point d'ancrage comporte un palan.

10. Dispositif selon l'une quelconque des revendications précédentes, dans lequel l'articulation est un genou, le segment supérieur est formé d'un fémur et le segment inférieur est formé du tibia et de la patella.

11. Produit programme d'ordinateur, chargeable directement dans la mémoire interne d'un ordinateur, comprenant des portions de code de logiciel pour l'exécution des étapes d'un procédé de contrôle d'un dispositif d'assistance à la mobilisation d'une articulation selon l'une quelconque des revendications précédentes, lorsque ledit programme est exécuté sur un ordinateur.

12. Produit programme d'ordinateur selon la revendication précédente, dans lequel le procédé de contrôle comporte une étape d'estimation du couple articulaire à partir d'informations provenant de capteurs équipant le dispositif, et une étape de calcul et d'application d'un couple d'assistance, correspondant à un pourcentage d'assistance prédéterminé.

13. Produit programme d'ordinateur selon la revendication précédente, dans lequel le procédé de contrôle comporte en outre une étape de filtrage de type passe bas des informations provenant des capteurs.

14. Produit programme d'ordinateur selon l'une quelconque des revendications 9 à 11, lorsque le dispositif à la mobilisation de l'articulation est réalisé selon la revendication 2, dans lequel la loi de commande du dispositif est déterminée à partir d'une trajectoire prédéterminée.

15. Produit programme d'ordinateur selon la revendication précédente, dans lequel le procédé de contrôle comporte un suivi de l'évolution de l'écart de position en valeur absolue entre d'une part la position réelle du segment inférieur à un instant donné et d'autre part la consigne de position de la trajectoire déterminée audit instant donné, et une application par les actionneurs du dispositif, d'une part d'une force réactive lorsque ledit écart augmente et d'autre part d'une force active lorsque ledit écart diminue.

## Patentansprüche

1. Vorrichtung (100) zum Unterstützen der Mobilisierung eines Gelenks (3), das ein oberes Segment (4) und ein unteres Segment (5) einer Person (1) verbindet, umfassend:
- ein erstes flexibles Führungselement (102), das so konfiguriert ist, dass es auf oder in der Nähe eines der Segmente (4) positioniert werden kann, wobei das erste Führungselement ein erstes Paar Kabeldurchgänge aufweist, das an dem ersten Führungselement angebracht ist, wobei ein Kabeldurchgang (P112) bzw. der andere Kabeldurchgang (P122) des ersten Paars Kabeldurchgänge des ersten Führungselements auf der Seite der Rückseite (52) bzw. auf der Seite der Vorderseite (54) des Segments angeordnet ist, wenn die Person das erste Führungselement trägt,
- ein zweites flexibles Führungselement (104), das so konfiguriert ist, dass es auf oder in der Nähe des anderen der Segmente (5) positioniert werden kann, wobei das zweite Führungselement ein erstes Paar Kabeldurchgänge aufweist, das an dem zweiten Führungselement angebracht ist, wobei ein Kabeldurchgang (P114) bzw. der andere Kabeldurchgang (P124) des ersten Paars Kabeldurchgänge des zweiten Führungselements auf der Seite der Rückseite (52) bzw. auf der Seite der Vorderseite (54) des Segments angeordnet ist, wenn die Person das zweite Führungselement trägt, wobei das zweite Führungselement ferner ein Paar Verankerungspunkte umfasst, wobei ein Verankerungspunkt (A11) bzw. der andere Verankerungspunkt (A12) des Paars Verankerungspunkte auf der Seite der Vorderseite (54) bzw. auf der Seite der Rückseite (52) des Segments angeordnet ist, wenn die Person das zweite Führungselement trägt,
- ein flexibles Trägerteil, das angeordnet ist, von der Person getragen zu werden und auf dem ein Paar Betätigungselemente (M11, M12) und ein Paar Wickler angeordnet sind, wobei jedes der Betätigungselemente des Paars Betätigungselemente einem Wickler des Paars Wickler zugeordnet und so angeordnet ist, dass es den zugehörigen Wickler in der Aufwickelrichtung und der Abwickelrichtung dreht,
- ein Paar Kabel (C11, C12), das ein erstes Kabel (C11) bzw. ein zweites Kabel (C12) umfasst und sich von einem der Wickler bzw. dem anderen der Wickler des Paars Wicklers bis zu einem der Verankerungspunkte bzw. dem anderen der Verankerungspunkte des zweiten Führungselements erstreckt, die einerseits durch einen Kabeldurchgang bzw. den anderen Kabeldurchgang des ersten Paars Kabeldurchgänge des ersten Führungselements und andererseits durch einen Kabeldurchgang bzw. den anderen Kabeldurchgang des ersten Paars Kabeldurchgänge des zweiten Führungselements verlaufen,
- eine Steuerung, die so konfiguriert ist, dass sie jedes der Betätigungselemente des Paars Betätigungselemente unabhängig voneinander steuert,
wobei:
- das erste flexible Führungselement (102) ferner ein zweites Paar Kabeldurchgänge umfasst, die an dem ersten Führungselement befestigt sind, wobei ein Kabeldurchgang (P212) bzw. der andere Kabeldurchgang (P222) des zweiten Paars Kabeldurchgänge des ersten Führungselements auf der Seite der Rückseite (52) bzw. auf der Seite der Vorderseite (54) des Segments angeordnet ist, wenn die Person das erste Führungselement trägt,
- das flexible zweite Führungselement (104) ferner ein zweites Paar Kabeldurchgänge umfasst, die an dem zweiten Führungselement befestigt sind, wobei ein Kabeldurchgang (P214) bzw. der andere Kabeldurchgang (P224) des zweiten Paars Kabeldurchgänge des zweiten Führungselements auf der Seite der Rückseite (52) bzw. auf der Seite der Vorderseite (54) des Segments angeordnet ist, wenn die Person das zweite Führungselement trägt, wobei das zweite Führungselement ferner ein zweites Paar Verankerungspunkte umfasst, wobei ein Verankerungspunkt (A21) bzw. der andere Verankerungspunkt (A22) des zweiten Paars Verankerungspunkte auf der Seite der Vorderseite (54) bzw. auf der Seite der Rückseite (52) des unteren Segments (5) der Person (1) angeordnet ist, wenn die Person das zweite Führungselement trägt,
wobei die Vorrichtung ferner Folgendes umfasst:
- ein zweites Paar Betätigungselemente (M21, M22) und ein zweites Paar Wickler, wobei jedes Betätigungselement des zweiten Paars Betätigungselemente einem Wickler des zweiten Paars Wickler zugeordnet und so angeordnet ist, dass es den zugeordneten Wickler in der Aufwickelrichtung und der Abwickelrichtung dreht, wobei die zweiten Paare Betätigungselemente und Wickler am flexiblen Trägerteil befestigt sind,
- ein zweites Paar Kabel (C21, C22), das ein erstes Kabel (C21) bzw. ein zweites Kabel (C22) umfasst und sich von einem der Wickler bzw. dem anderen der Wickler des zweiten Paars Wickler zu einem der Verankerungspunkte bzw. dem anderen der Verankerungspunkte des zweiten Führungselements erstreckt, die einerseits durch einen Kabeldurchgang bzw. den anderen Kabeldurchgang des zweiten Paars Kabeldurchgänge des ersten Führungselements und andererseits durch einen Kabeldurchgang bzw. den anderen Kabeldurchgang des zweiten Paars Kabeldurchgänge des zweiten Führungselements verlaufen,
wobei die Steuerung ferner so konfiguriert ist, dass sie jedes Betätigungselement des zweiten Paars Betätigungselemente unabhängig voneinander steuert.

2. Vorrichtung nach dem vorhergehenden Anspruch, wobei die Steuerung so konfiguriert ist, dass sie die Betätigungselemente des Paars Betätigungselemente gemäß einem vorgegebenen Steuerbefehl steuert.

3. Vorrichtung nach dem vorhergehenden Anspruch, wobei, wenn das erste Verankerungselement und das zweite Verankerungselement an der Person angebracht sind, jedes der Kabel einen Markierungspunkt aufweist, der als der Punkt bestimmt wird, der einer geschätzten Position der Drehachse des Gelenks am nächsten liegt, und die durch die beiden Markierungspunkte (PR11, PR12) des ersten Paars Kabel gebildete Richtung und die durch die beiden Markierungspunkte (PR21, PR22) gebildete Richtung des zweiten Paars Kabel im Wesentlichen orthogonal sind.

4. Vorrichtung nach dem vorhergehenden Anspruch, wobei die Richtungen mit der Querachse und der anteroposterioren Achse einen Winkel von 45° bilden.

5. Vorrichtung nach einem der vorhergehenden Ansprüche, die mehr als zwei Paare Kabel umfasst.

6. Vorrichtung nach einem der vorhergehenden Ansprüche, die ein Mittel zur Bestimmung des Drehmoments des Gelenks umfasst.

7. Vorrichtung nach einem der vorhergehenden Ansprüche, die ein Mittel zur Bestimmung der Ausrichtung des oberen Segments und/oder des unteren Segments im Bezug zur Längsachse umfasst.

8. Vorrichtung nach einem der vorhergehenden Ansprüche, die ein Mittel zur Erfassung einer Auflage des unteren Segments auf einem Boden oder einem Rahmen umfasst.

9. Vorrichtung nach einem der vorhergehenden Ansprüche, wobei eine Übertragungskette zwischen einem Motor und einem Verankerungspunkt ein Hebezeug umfasst.

10. Vorrichtung nach einem der vorhergehenden Ansprüche, wobei das Gelenk ein Kniegelenk ist, das obere Segment durch einen Oberschenkel und das untere Segment durch das Schienbein und die Kniescheibe gebildet wird.

11. Computerprogrammprodukt, das direkt in den internen Speicher eines Computers geladen werden kann und Teile eines Softwarecodes zum Ausführen der Schritte eines Verfahrens zum Steuern einer Hilfsvorrichtung zur Mobilisierung eines Gelenks nach einem der vorhergehenden Ansprüche umfasst, wenn das Programm auf einem Computer ausgeführt wird.

12. Computerprogrammprodukt nach dem vorhergehenden Anspruch, wobei das Steuerverfahren einen Schritt des Schätzens des Drehmoments des Gelenks anhand von den Informationen umfasst, die von den Sensoren stammen, mit denen die Vorrichtung ausgestattet ist, und einen Schritt des Berechnens und des Anwendens eines Unterstützungsdrehmoments, das einem vorgegebenen Unterstützungsprozentsatz entspricht.

13. Computerprogrammprodukt nach dem vorhergehenden Anspruch, wobei das Steuerverfahren ferner einen Schritt des Tiefpassfilterns der von den Sensoren stammenden Informationen umfasst.

14. Computerprogrammprodukt nach einem der Ansprüche 9 bis 11, wenn die Vorrichtung zur Mobilisierung des Gelenks nach Anspruch 2 ausgebildet ist, wobei der Steuerbefehl der Vorrichtung anhand einer vorgegebenen Trajektorie bestimmt wird.

15. Computerprogrammprodukt nach dem vorhergehenden Anspruch, wobei das Steuerverfahren das Überwachen des Verlaufs der Positionsabweichung in absoluten Werten zwischen der tatsächlichen Position des unteren Segments zu einem bestimmten Zeitpunkt einerseits und dem Positionssollwert der bestimmten Trajektorie zu dem bestimmten Zeitpunkt andererseits und das Anwenden einer reaktiven Kraft durch die Betätigungselemente der Vorrichtung einerseits, wenn diese Abweichung zunimmt, und einer aktiven Kraft andererseits, wenn diese Abweichung abnimmt, umfasst.

## Claims

1. A device (100) for assisting with mobilising a joint (3) connecting an upper segment (4) and a lower segment (5) of a person (1), including:
- a first flexible guiding element (102) configured to be positioned on or in the vicinity of one of the segments (4), the first guiding element having a first pair of cable passages attached to said first guiding element, one cable passage (P112), respectively the other cable passage (P122), of the first pair of cable passages being disposed on the side of the posterior face (52), respectively on the side of the anterior face (54), of said segment when the person wears the first guiding element,
- a second flexible guiding element (104) configured to be positioned on or in the vicinity of the other of the segments (5), the second guiding element having a first pair of cable passages attached to said second guiding element, one cable passage (P114), respectively the other cable passage (P124), of the first pair of cable passages being disposed on the side of the posterior face (52), respectively on the side of the anterior face (54), of said segment when the person wears the second guiding element, the second guiding element further including a pair of anchoring points, one anchoring point (A11), respectively the other anchoring point (A12), of the pair of anchoring points being disposed on the side of the anterior face (54), respectively on the side of the posterior face (52), of said segment, when the person wears the second guiding element,
- a flexible support part arranged to be worn by the person and whereon a pair of actuators (M11, M12) and a pair of winders are disposed, each of the actuators of the pair of actuators being associated with a winder of the pair of winders and being arranged to turn the associated winder in the winding direction and the unwinding direction,
- a pair of cables (C11, C12) including a first cable (C11), respectively a second cable (C12) extending from one of the winders, respectively the other of the winders, of the pair of winders, to one of the anchoring points, respectively the other of the anchoring points, of the second guiding element, passing, on one hand, through one cable passage, respectively the other cable passage, of the first pair of cable passages of the first guiding element and, on the other, through one cable passage, respectively the other cable passage, of the first pair of cable passages of the second guiding element,
- a controller configured to control each of the actuators of the pair of actuators independently.
wherein:
- the first flexible guiding element (102) further includes a second pair of cable passages attached to said first guiding element, one cable passage (P212), respectively the other cable passage (P222), of said second pair of cable passages of the first guiding element being disposed on the side of the posterior face (52), respectively on the side of the anterior face (54), of said segment when the person wears the first guiding element,
- the second flexible guiding element (104) further includes a second pair of cable passages attached to said second guiding element, one cable passage (P214), respectively the other cable passage (P224), of said second pair of cable passages of the second guiding element being disposed on the side of the posterior face (52), respectively on the side of the anterior face (54), of said segment when the person wears the second guiding element, the second guiding element further including a second pair of anchoring points, one anchoring point (A21), respectively the other anchoring point (A22), of the second pair of anchoring points being disposed on the side of the anterior face (54), respectively on the side of the posterior face (52), of the lower segment (5) of the person (1) when the person wears the second guiding element,
the device further including:
- a second pair of actuators (M21, M22) and a second pair of winders, each of the actuators of the second pair of winders being associated with a winder of the second pair of winders and being arranged to turn the associated winder along the winding direction and the unwinding direction, the second pairs of actuator and winder being attached to the flexible support part,
- a second pair of cables (C21, C22) including a first cable (C21), respectively a second cable (C22) extending from one of the winders, respectively the other of the winders, of the second pair of winders, to one of the anchoring points, respectively the other of the anchoring points, of the second guiding element, passing, on one hand, through one cable passage, respectively the other cable passage, of the second pair of cable passages of the first guiding element and, on the other, through one cable passage, respectively the other cable passage, of the second pair of cable passages of the second guiding element,
a controller being furthermore configured to control each of the actuators of the second pair of actuators independently.

2. The device according to the preceding claim, wherein the controller is configured to control the actuators of the pair of actuators according to a predetermined control law.

3. The device according to the preceding claim, wherein, when the first anchoring element and the second anchoring element are disposed on the person, each of the cable has a remarkable point, determined as the closest point to an estimated position of the axis of rotation of the joint, and the direction formed by the two remarkable points (PR11, PR12) of the first pair of cables and the direction formed by the two remarkable points (PR21, PR22) of the second pair of cables are substantially orthogonal.

4. The device according to the preceding claim, wherein, the directions form an angle of 45° with the transverse axis and the anteroposterior axis.

5. The device according to any one of the preceding claims, including more than two pairs of cables.

6. The device according to any one of the preceding claims, including a means for determining the joint torque.

7. The device according to any one of the preceding claims, including a means for determining the orientation of the upper segment and/or the lower segment with respect to the longitudinal axis.

8. The device according to any one of the preceding claims, including a means for detecting a bearing of the lower segment on a floor or a frame.

9. The device according to any one of the preceding claims, wherein a power transmission chain between a motor and an anchoring point includes a hoist.

10. The device according to any one of the preceding claims, wherein the joint is a knee, the upper segment is formed from a femur and the lower segment is formed from the tibia and the patella.

11. A computer program product, loadable directly in the internal memory of a computer, comprising software code portions for executing the steps of a method for controlling a device for assisting with mobilising a joint according to any one of the preceding claims, when said program is executed on a computer.

12. The computer program product according to the preceding claim, wherein the control method includes a step of estimating the joint torque based on information from sensors equipping the device, and a step of computing and applying an assistance torque, corresponding to a predetermined assistance percentage.

13. The computer program product according to the preceding claim, wherein the control method further includes a step of low-pass type filtering of the information from the sensors.

14. The computer program product according to any one of claims 9 to 11, when the device for mobilising the joint is embodied according to claim 2, wherein the control law of the device is determined using a predetermined trajectory.

15. The computer program product according to the preceding claim, wherein the control method includes tracking of the progression of the position deviation in absolute values between, on the one hand, the actual position of the lower segment at a given time and, on the other hand, the position set-point of the determined trajectory at said given time, and an application by the actuators of the device, on one hand of a reactive force when said deviation increases and, on the other of an active force when said deviation decreases.
